# EUROPÄISCHE PATENTSCHRIFT

(11) **EP 0 643 065 B1**
(45) Veröffentlichungstag und Bekanntmachung des Hinweises auf die Patenterteilung: **10.11.1999**
(21) Anmeldenummer: 94113441.3
(22) Anmeldetag: 29.08.1994
(51) Int. Cl.: C07F 9/50, C07F 15/00, C07F 9/655, C07F 9/53, C07C 51/373, C07M 7/00

(54) **Neue Bisphosphine für asymmetrische Hydrierkatalysatoren**
Novel bisphosphines for asymetric hydrogenation catalysts
Nouvelles bisphosphines pour catalyseurs d'hydrogénation asymétrique

(30) Priorität: 10.09.1993 DE 4330730
(43) Veröffentlichungstag der Anmeldung: 15.03.1995
(73) Patentinhaber: BAYER AG, 51368 Leverkusen (DE)
(72) Erfinder: Laue, Dr. Christian, D-40789 Monheim (DE); Schröder, Dr. Georg, D-51375 Leverkussen (DE); Arlt, Prof. Dr. Dieter, D-51065 Köln (DE); Grosser, Dr. Rolf, D-51373 Leverkussen (DE)

(56) Entgegenhaltungen:
- EP-A- 0 104 375
- EP-A- 0 398 132
- Synlett, 1991, Seiten 827-829

## Beschreibung

Die Erfindung betrifft neue enantiomerenreine Bisphosphine, Verfahren zu ihrer Herstellung und ihre Verwendung in Metallkomplexen als Katalysatoren für asymmetrische Hydrierungen.

Es ist bereits bekannt, Komplexe von bestimmten Bisphosphinen mit Metallen der Gruppe VIII für asymmetrische Hydrierungen und enantioselektive Wasserstoffverschiebungen einzusetzen (vgl. EP-A 398 132 und R. Noyori, Modern Synthetic Methods, 113-198 (1989)). Die erfindungsgemäßen Phosphine und die aus ihnen hergestellten Komplexe unterscheiden sich in ihrer chemischen Struktur eindeutig von den bisher bekannten Verbindungen. Es konnte nicht erwartet werden, daß durch die erfindungsgemäße Änderung der Substituenten Bisphosphine bzw. Bisphosphinruthenium-Komplexe erhalten werden mit denen sich die obengenannten enantioselektiven Reaktionen mit höherer Enantioselektivität durchführen lassen als mit den bekannten Bisphosphinkomplexen. Auch in EP-A 529 444 wird z.B. die enantioselektive Hydrierung von 2-(3-Benzyl-phenyl)-propensäure zur entsprechenden Propansäure mit einem Ruthenium-Bisphosphin-Komplex (BINAP-Komplex) mit einer maximalen Enantioselektivität von 80 % e.e. beschrieben. Die erfindungsgemäßen Rutheniumkomplexe liefern bei der entsprechenden enantioselektiven Hydrierung von Propensäurederivaten eine signifikantere Enantioselektivität. So ergibt sich z.B. beim Einsatz des Rutheniumkomplexes mit dem Bisphosphinfuranderivat eine Enantioselektivität von ca. 88 % e.e. Die erfindungsgemäßen Verbindungen der Formel (I) und die mit ihnen gebildeten Komplexe sind somit besonders geeignet spezielle asymmetrische Hydrierungen in hohen Ausbeuten und guter Enantioselektivität durchzuführen.

Die Erfindung betrifft enantiomerenreine Bisphosphine der allgemeinen Formel (I) 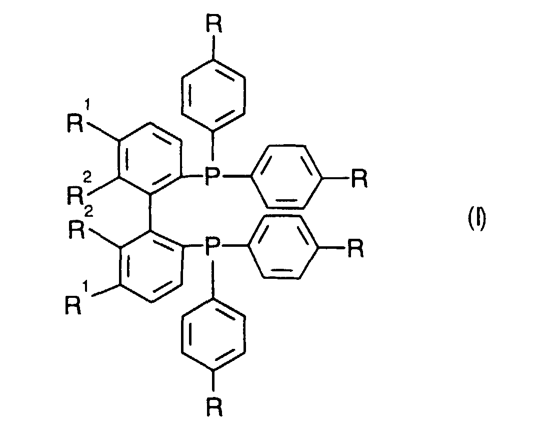 in welcher
- R: für Wasserstoff oder Alkyl mit 1 bis 4 C-Atomen steht,
- R¹: für Wasserstoff steht und
- R²: für Chlor steht oder
- R¹ und R²: gemeinsam für den Rest der Formel 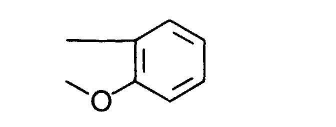 stehen.

Besonders bevorzugt sind Bisphosphine der allgemeinen Formel (I), in welcher
- R: für Wasserstoff steht und
- R¹ und R²: die oben angegebene Bedeutung haben.

Die Bisphosphine der allgemeinen Formel (I) können hergestellt werden, indem man 3-Halogenphenylverbindungen der allgemeinen Formel (II) 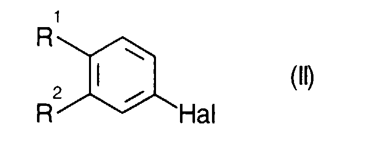 in welcher
- R¹ und R²: die oben angegebene Bedeutung haben und
- Hal: für Halogen, insbesondere für Brom steht,
mit Diphenylphosphoroxychlorid der allgemeinen Formel (III) 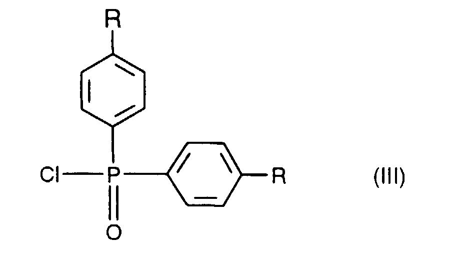 in welcher
- R: die oben angegebene Bedeutung hat,
nach üblichen Methoden z.B. über eine Grignard-Reaktion zu Verbindungen der allgemeinen Formel (IV) 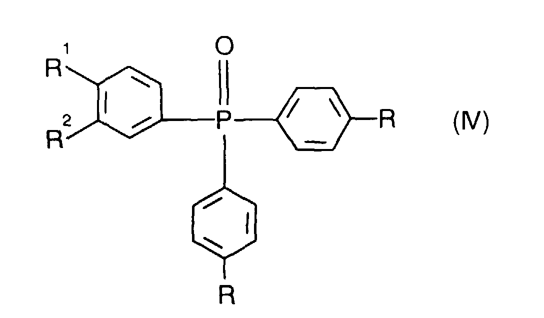 in welcher
- R, R¹ und R²: die oben angegebene Bedeutung haben,
umsetzt und diese mit Lithium in ortho-Position metalliert und anschließend mit Iod zu Verbindungen der Formel (V) 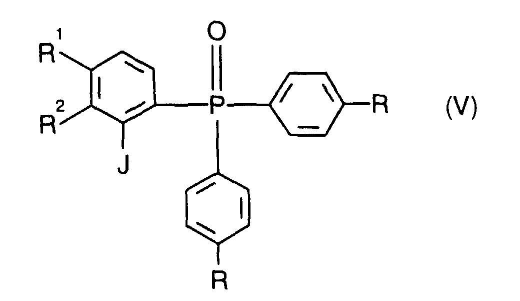 umsetzt, nach üblichen Methoden (Ullmann-Kupplung) zu racemischen Verbindungen der allgemeinen Formel (VI) 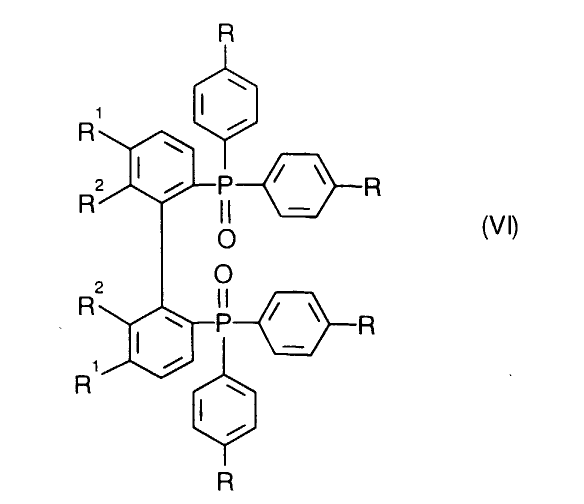 in welcher
- R, R¹ und R²: die oben angegebene Bedeutung haben,
dimerisiert, und diese Phosphinoxide in ihre Enantiomere auftrennt, z.B. durch präparative Chromatographie an chiralen Phasen oder durch Kristallisationsverfahren mit optisch aktiven Säuren und anschließend die erhaltenen (S)- oder (R)-Enantiomeren nach üblichen Methoden zu Verbindungen der Formel (I) reduziert.

Bevorzugt verwendet man als chirale Phase optisch aktive Polymere von optisch aktiven (Meth)acrylsäure-Derivaten. Besonders bevorzugt sind hier Polymerisate von optisch aktiven N-(Meth)acryloyl-aminosäure-Derivaten, wie sie in EP-379 917 beschrieben sind. Ganz besonders bevorzugt seien hier Polymerisate aus folgenden optisch aktiven N-Methacryloyl-aminosäureamiden genannt: N-Methacryloyl-L- bzw. -D-aminosäurementhylamide, wobei als Aminosäure z.B. Alanin, Leucin, Valin oder sonstige Aminosäuren in Frage kommen.

Als Fließmittel für die Trennung der Racemate werden übliche organische Lösemittel bzw. Lösemittelgemische verwendet, die das als Adsorbens eingesetzte Polymerisat anquellen und das zu trennende Racemat lösen. Beispielsweise seien genannt: Kohlenwasserstoffe wie Benzol, Toluol oder Xylol, Ether wie Diethylether, Dioxan, Tetrahydrofuran, Halogenkohlenwasserstoffe wie Di- oder Trichlormethan, Aceton, Acetonitril, Alkohole wie Ethanol oder Propanol oder Essigester oder aber Gemische der genannten Lösemittel. Als besonders geeignet haben sich Mischungen aus Toluol und Tetrahydrofuran sowie Toluol und Dioxan erwiesen.

Die Erfindung betrifft auch die neuen Zwischenprodukte der allgemeinen Formel (V) und der Formel (VI), wobei die Substituentenbedeutungen jeweils den für die Formel (I) angegebenen Bedeutungen entsprechen, sowie die Verfahren zur Herstellung dieser Zwischenprodukte.

Die erfindungsgemäßen Phosphorverbindungen der Formel (I) bilden Komplexe mit Übergangsmetallen wie etwa Metallen der Gruppe VIII, insbesondere mit Ruthenium, Rhodium und Iridium, welche als Katalysatoren bei asymmetrischen Hydrierungen und auch für enantioselektive Wasserstoffverschiebungen in prochiralen, allylischen Systemen verwendbar sind. Für die erwähnten Hydrierungen sind Ruthenium-, Iridium- und Rhodium-Komplexe bevorzugt, während für Isomerisierungen Rhodium-Komplexe bevorzugt sind. Diese Katalysatoren, d.h. die Komplexe aus einem Metall der Gruppe VIII und den Phosphorverbindungen der Formel (I) sind neu und ebenfalls Gegenstand der vorliegenden Erfindung.

Die in Frage stehenden Komplexe können in an sich bekannter Weise hergestellt werden, z.B. indem man eine Verbindung der Formel (I) mit einer Verbindung, welche ein Metall der Gruppe VIII abgeben kann, in einem geeigneten, inerten organischen oder wäßrigen Lösungsmittel umsetzt. Als geeignete, z.B. Rhodium abgebende Verbindungen können beispielsweise genannt werden, organische Rhodium-Komplexe mit Ethylen, Propylen und dergleichen, sowie mit bis-Olefinen, z.B. 1,5-Cyclooctadien, 1,5-Hexadien, Bicyclo(2.2.1)hepta-2,5-dien oder mit weiteren Dienen, welche mit Rhodium leicht lösliche Komplexe bilden. Bevorzugte, Rhodium abgebende Verbindungen sind z.B. Di-chloro-bis-(1,5-cyclooctadien)dirhodium, Di-chloro-bis(-norbornadien)dirhodium, Bis-(1,5-cyclooctadien)-rhodium-tetrafluorborat oder Bis-(cyclooctadien)rhodium-perchlorat. Als Iridium abgebende Verbindung kann beispielsweise Di-chloro-bis-(1,5-cyclooctadien)diiridium genannt werden.

Von besonderem Interesse sind Rutheniumkomplexe mit Bisphosphinen der allgemeinen Formel (I). Als typisch aber nicht als Einschränkung seien die Rutheniumkomplexe der folgenden Formeln (VII) bis (XI) genannt.

### Beispiele für typische Rutheniumkomplexe

Ru₂Cl₄B₂(S) (VII)

[Ru Hal Q B]^{⊕} y ^{⊖} (VIII)

Ru Bₙ OOCR³OOCR⁴ (IX)

[Ru Hₓ Bₙ]^{m⊕} Yₘ ^{⊖} (X)

[Ru Hal (PR⁵ ₂R⁶)B]⁽²⁺⁾ Hal₂ ^{⊖} (XI)

[Ru H Hal B₂] (XII)

[B Ru (acac)₂] (XIII)

worin:
- acac: Acetylacetonat
- B: für ein Bisphosphin der allgemeinen Formel (I) steht,
- Hal: für Halogen, insbesondere für Iod, Chlor oder Brom steht,
- R³ und R⁴: gleich oder verschieden sind und für Alkyl mit bis zu 9 C-Atomen, vorzugsweise bis zu 4 C-Atomen, welches gegebenenfalls substituiert ist durch Halogen, insbesondere Fluor, Chlor oder Brom oder für Phenyl stehen, welches gegebenenfalls durch Alkyl mit 1 bis 4 C-Atomen substituiert ist oder für eine α-Aminoalkylsäure mit vorzugsweise bis zu 4 C-Atomen stehen,
oder
gemeinsam eine Alkylidengruppe mit bis zu 4 C-Atomen bilden,
- R⁵ und R⁶: jeweils gleich oder verschieden sind und für gegebenenfalls substituiertes Phenyl stehen, vorzugsweise substituiert durch Alkyl mit 1 bis 4 C-Atomen oder Halogen,
- Y: für Cl, Br, J, ClO₄, BF₄ oder PF₆ steht,
- Q: für einen unsubstituierten oder substituierten Benzolring wie p-Cymol steht,
- S: für ein tertiäres Amin wie z.B. Triethylamin, Tri-n-Butylamin oder Pyridin steht,
- n und m: stehen jeweils für 1 oder 2,
- x: steht für 0 oder 1,
wobei in Formel (X) n für 1 und m für 2 steht, wenn x = 0 bedeutet,
und n für 2 und m für 1 steht, wenn x = 1 bedeutet.

Die Komplexe der Formeln (VII) bis (XIII) können nach an sich bekannten Methoden hergestellt werden.

Die Komplexe der Formeln (VII) und (XII) lassen sich z.B. in analoger Weise gemäß den Verfahren herstellen, die in EP-174 057 oder in Chem. Comm. 922 (1985) beschrieben werden.

Die Komplexe der allgemeinen Formel (VIII) ergeben sich z.B. durch Umsetzung von bekannten Rutheniumkomplexen [RuHal₂Q]₂ mit Bisphosphinen der allgemeinen Formel (I) in inerten organischen Lösungsmitteln, wie z.B. in EP 366 390 beschrieben.

Komplexe der allgemeinen Formel (IX), n = 1 können z.B. erhalten werden nach Verfahren, die in EP 245 959 angegeben sind, indem man Komplexe der allgemeinen Formel (VII) mit entsprechenden Carbonsäuren, vorzugsweise in alkoholischen Lösungsmitteln umsetzt.

Komplexe der Formeln (IX) n = 2 und mit n = 1 und R³, R⁴ = CF₃ können nach den in EP 272 787 angegebenen Verfahren hergestellt werden.

Die Komplexe der allgemeinen Formel (X) können hergestellt werden analog dem Verfahren gemäß EP-256 634.

Die Komplexe der allgemeinen Formel (XI) können hergestellt werden analog dem Verfahren gemaß EP-470 756 durch Umsetzung der dort beschriebenen Ru-Vorstufen mit den erfindungsgemäßen Bisphosphinen der allgemeinen Formel (I).

Komplexe der Formel (XIII) können analog den in P. Stahly et al., Organomettalics 1993, 1467 ff. angegebenen Verfahren dargestellt werden.

Die erfindungsgemäßen Bisphosphine in Form ihrer Komplexe mit Metallen der Gruppe VIII und insbesondere Ruthenium sind verwendbar für asymmetrische Hydrierungen. Geeignete Substrate sind substituierte oder unsubstituierte α- oder β-Ketoester oder α- oder β-Keto-Amide, α- oder β-Amino- oder α- oder β-Hydroxy-Ketone und Acetamidozimtsäurederivate.
Besonders geeignet sind 2-Arylpropensäuren wie z.B. 2-(6'-Methoxy-2'-naphthyl)propensäure, 2-(4-Isobutyl)-propensäure und 2-(3-Benzyl-phenyl)-propensäure und ihre Salze, z.B mit tertiären Aminen.

Bei der Durchführung derartiger Hydrierungen können diese Komplexe zuerst hergestellt und dann zu einer Lösung der zu hydrierenden Substanz gegeben werden. Alternativ können sie jedoch auch in situ hergestellt werden, z.B. auch in Gegenwart einer zu hydrierenden Substanz.

Die asymmetrische Hydrierung kann in einem geeigneten, unter den Reaktionsbedingungen inerten organischen Lösungsmittel erfolgen. Als derartige Lösungsmittel können insbesondere genannt werden, niedere Alkohole wie z.B. Methanol oder Ethanol, oder Gemische derartiger Alkohole mit halogenierten Kohlenwasserstoffen wie Methylenchlorid, Chloroform und dergleichen, oder mit cyclischen Ethern wie Tetrahydrofuran oder Dioxan, und dergleichen.

Das Verhältnis von den Metallen zu Bisphosphinen der allgemeinen Formel (I) liegt zweckmäßig zwischen etwa 0,5 und etwa 2 Mol, vorzugsweise bei etwa 1 Mol Ruthenium pro Mol Bisphosphin-Ligand. Das Verhältnis von Metall in den Komplexen zu den hydrierenden Substanzen liegt zweckmäßig zwischen etwa 0,0005 und 1 Mol-%, vorzugsweise zwischen etwa 0,005 und 0,6 Mol-%.

Die asymmetrische Hydrierung mit den erfindungsgemäßen Komplexen erfolgt zweckmäßig bei einer Temperatur von etwa 0°C bis etwa 100°C, in Abhängigkeit des verwendeten Substrates. Diese Hydrierung erfolgt zweckmäßig auch unter einem Druck, vorzugsweise bei einem Druck von etwa 5 bis etwa 200 bar, besonders bevorzugt von etwa 40 bis etwa 140 bar.

Weiterhin sind die erfindungsgemäßen Bisphosphinkomplexe als Katalysatoren verwendbar für enantioselektive Wasserstoffverschiebungen in prochiralen allylischen Systemen. Besonders interessant sind sie z.B. im Zusammenhang mit der Herstellung von optisch aktiven Verbindungen der allgemeinen Formel (XIV) 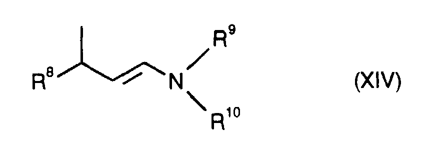 worin
- R⁸: geschütztes Hydroxymethyl oder einen Rest der Formel 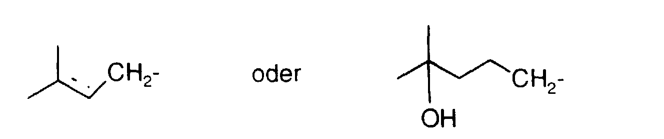 darstellt, worin die gestrichelte Linie eine zusätzliche Bindung darstellen kann, und
- R⁹ und R¹⁰: gleich oder verschieden sind und niederes Alkyl (1-7 C-Atome) bedeuten,
ausgehend von Verbindungen der allgemeinen Formel (XV) 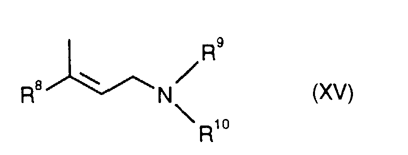 worin
- R⁸, R⁹ und R¹⁰: die obige Bedeutung haben.

Die Verbindungen (XIV) bzw. die daraus durch Hydrolyse erhaltenen Aldehyde, sowie die von letzteren abgeleiteten Säuren und Alkohole sind z.B. von Interesse als Zwischenprodukte bei der Synthese der Seitenketten der Vitamine E und K_{I}.

Zur Durchführung der erwähnten Wasserstoffverschiebungen können die Phosphorverbindungen der Formel (I) als solche in einer Lösung einer zu behandelnden Verbindung mit einer z.B. Rhodium oder Iridium abgebenden Verbindung in Kontakt gebracht werden. Andererseits können die Phosphorverbindungen der Formel (I) zunächst in einem geeigneten Lösungsmittel mit einer z.B. Rhodium oder Iridium abgebenden Verbindung zu dem entsprechenden Katalysator-Komplex umgesetzt werden, und dieser dann zu einer Lösung einer zu behandelnden Verbindung gegeben werden, wobei die letztere Methode bevorzugt ist.

Sowohl die Umsetzung der Phosphorverbindungen der Formel (I) mit einer z.B. Rhodium oder Iridium abgebenden Verbindung, wie auch die erwähnten Wasserstoffverschiebungen können in geeigneten, unter den Reaktionsbedingungen inerten organischen Lösungsmitteln durchgeführt werden. Als solche seien insbesondere genannt niedere Alkanole wie z.B. Methanol oder Ethanol, aromatische Kohlenwasserstoffe wie Benzol oder Toluol, cyclische Ether wie Tetrahydrofuran oder Dioxan, Ester wie z.B. Essigester, oder auch Gemische hiervon, und dergleichen. Weiterhin können die Komplexbildungen auch in wäßrigem Medium oder in Dichlormethan durchgeführt werden.

Das Verhältnis zwischen z.B. Rhodium oder Iridium und den Liganden der Formel (I) liegt zweckmäßig zwischen etwa 0,05 und etwa 5 Mol, vorzugsweise zwischen etwa 0,5 und etwa 2 Mol Metall pro Mol Ligand der Formel (I).

Die Menge an Metall, in den Komplexen mit den Liganden der Formel (I), bezogen auf die zwecks Wasserstoffverschiebung zu behandelnden Verbindungen, liegt vorzugsweise zwischen etwa 0,005 und etwa 0,5 Mol-%, insbesondere zwischen etwa 0,01 und etwa 0,2 Mol-%.

Die erwähnten Wasserstoffverschiebungen unter Verwendung von Metall-Komplexen mit den Liganden der Formel (I) können zweckmäßig in einem inerten, organischen Lösungsmittel und bei einer Temperatur von etwa Raumtemperatur bis etwa 130°C durchgeführt werden. Diese Reaktion erfolgt vorzugsweise bei erhöhter Temperatur, d.h. je nach verwendetem Lösungsmittel entweder bei Rückflußtemperatur des Reaktionsgemisches oder in einem geschlossenen Gefäß unter Druck.

### Ausführungsbeispiele:

Verwendete Abkürzungen:
cym (=Cymol) =p-Methyl-isopropylbenzol
THF = Tetrahydrofuran
DMF = Dimethylformamid
alle ³¹P-Spektren sind {¹H}-breitband entkoppelt

### A) Herstellung der Bisphosphine (I)

### 1) (R)-(-)- und (S)-(+)-(6,6'Dichlor-biphenyl-2,2'-diyl)-bis-diphenylphosphin

### a) (3-Chlor-phenyl)-diphenylphosphinoxid (IV)

Zu einer siedenden Mischung aus 1,27 g Mg und 10 ml THF wird eine Lösung von 10 g 3-Brom-chlorbenzol in 30 ml THF gegeben und noch 1 Stunde unter Rückfluß gekocht. Anschließend wird eine Lösung von 12,4 g Diphenylphosphinoxychlorid in 30 ml Toluol bei 0°C zugetropft und noch 1 h bei Raumtemperatur gerührt.
Nach Neutralisation der Reaktionslösung mit 2N HCl wird mit 150 ml Essigester und Wasser versetzt und die organische Phase wird getrocknet und eingeengt. Man erhält 8,82 g (54 % d.Th.) gelbe Kristalle.
Fp.: 108-110°C

### b) (3-Chlor-2-iod-phenyl)-diphenylphosphinoxid (V)

8,81 g (3-Chlor-phenyl)-diphenylphosphinoxid in 70 ml THF wird mit 14,5 ml einer 2M Lösung von Lithium-diisopropylamid in THF/n-Heptan versetzt und noch 10 min bei -76°C gerührt. Bei -76°C wird eine Lösung von 7,83 g Iod in 30 ml THF zugegeben.
Anschließend wird mit 2N HCl hydrolysiert und mit Essigester extrahiert. Die organische Phase wird über Kieselgel filtriert, getrocknet und eingeengt. Es bleiben 11,7 g (Ausbeute: 95 % d.Th.) Rohprodukt, das direkt gemäß c) weiter eingesetzt wird.

### c) racemisches (6,6'-Dichlor-biphenyl-2,2'-diyl)-bis-diphenylphosphinoxid (VI)

Eine Mischung aus 11,4 g (3-Chlor-2-iod-phenyl)-diphenylphosphinoxid, 5,00 g Cu-Pulver und 55 ml DMF werden 3 Tage bei 140°C intensiv unter Luftausschluß gerührt. Die erkaltete Reaktionslösung wird vom Lösungsmittel befreit und über Kieselgel chromatographiert (Laufmittel: Essigester in Cyclohexan; 25 bis 75 %).
Die Hauptfraktion wird in tert.-Butylmethylether ausgerührt.
Es bleiben 5,42 g Kristalle (67 % Ausbeute).
³¹P-NMR ([D₆]-DMSO): 28,0 ppm

### d) Enantiomeren-Trennung

1 g racemisches (6,6'-Dichlor-biphenyl-2,2'-diyl)-bis-diphenylphosphinoxid aus Beispiel 1c), gelöst in 100 ml THF wird auf eine Glassäule (10 cm, Länge 100 cm) mit gequollenem Perlpolymerisat aus N-(Methacryloyl-L-alanin-1-menthylamid) (EP-379 917) aufgegeben und mit Toluol/THF 3:1 (v/v) bei einem Fluß von 10 ml/min eluiert. Nach 15 h eluiert das erste Enantiomer. Die fraktionierten Eluate werden nach analytischer Kontrolle auf Enantiomerenreinheit vereinigt. Nach üblicher Aufarbeitung erhält man 0,4 g des zuerst eluierten (+)-Enantiomeren und 0,35 g des entsprechenden (-)-Enantiomeren.
(R)-(+)-(6,6'-Dichlorbiphenyl-2,2'-diyl)-bis-diphenylphosphinoxid (VI)
[α]_{D} = +46° (c = 1, DMF)
Fp.: ab 270°C Zersetzung
(S)-(-)-(6,6'-Dichlorbiphenyl-2,2'-diyl)-bis-diphenylphosphinoxid (VI)
[α]_{D} = -46° (c = 1, DMF)
Fp.: ab 270°C Zersetzung

### e) (S)-(+)-(6,6'-Dichlor-biphenyl-2,2'-diyl)-bis-diphenylphosphin (I)

Eine Mischung aus 860 mg (S)-(-)-(6,6'-Dichlor-biphenyl-2,2'-diyl)-bis-diphenylphosphinoxid, 3 ml Tributylamin, 40 ml Xylol und 0,81 ml Trichlorsilan werden 16 h am Rückfluß gekocht. Bei 0°C werden anschließend 13 ml 30 % NaOH zugegeben. Die Mischung wird mit Methylenchlorid extrahiert. Nach Trocknung mit gesättigter Natriumchloridlösung und MgSO₄ wird durch Chromatographie an Kieselgel (10 bis 40 % Essigester in Cyclohexan) gereinigt.
Ausbeute: 670 mg (82 % der Theorie), Fp. = 230-235°C
[α]_{D} = +51° (c = 1, CHCl₃)

### f) (R)-(-)-(6,6'-Dichlor-biphenyl-2,2'-diyl)-bis-diphenylphosphin (I)

Die Reaktion wurde analog e) mit 916 mg (R)-(+)-(6,6'-Dichlorbiphenyl-2,2'-diyl)-bis-diphenylphosphinoxid durchgeführt:
Ausbeute: 710 mg (82 % der Theorie)
Fp.: 230-235°C
[α]_{D} = -50° (c = 1, CHCl₃)

### 2) (R)- und (S)-[Bis-4,4'-dibenzofuran-3,3'-diyl]-bis(diphenylphosphin)

### a) (Dibenzofuran-3-yl)-diphenylphosphinoxid (IV)

Zu einer siedenden Mischung aus 4,31 g Mg und 10 ml THF wird eine Lösung von 25 g 3-Brom-dibenzofuran in 90 ml THF gegeben und noch 1 Stunde unter Rückfluß gekocht. Anschließend wird eine Lösung von 23,9 g Diphenylphosphinoxychlorid in 45 ml THF bei 0°C zugetropft und noch 1 h bei Raumtemperatur gerührt.
Nach Neutralisation der Reaktionslösung mit 1N HCl wird mit 150 ml Essigester und Wasser versetzt und die organische Phase mit MgSO₄ getrocknet und eingeengt. Der Rückstand wird mit tert.-Butyl-methylether 16 h gerührt und filtriert. Als Rückstand bleiben 24,9 g gelbe Kristalle (65 % d.Th.).
Fp.: 151-155°C

### b) (4-Iod-dibenzofuran-3-yl)-diphenylphosphinoxid (V)

9,8 g (Dibenzofuran-3-yl)-diphenyl-phosphinoxid in 100 ml THF wird mit 13,3 ml einer 2M Lösung von Lithium-diisopropylamid in THF/n-Heptan versetzt und noch 10 min bei -78°C gerührt. Bei -76°C wird eine Lösung von 7,9 g Iod in 30 ml THF zugegeben.
Anschließend wird mit 2N HCl hydrolysiert, Essigester zugesetzt, mit Natriumthiosulfat-Lösung, Wasser und gesättigter NaCl-Lösung gewaschen, mit MgSO₄ getrocknet und eingeengt. Es bleiben 12,7 g (98 % d.Th.) Rohprodukt.
Reinheit (HPLC): 94 %
Fp.: 288 bis 294°C (gereinigtes Produkt)

### c) rac.-[Bis-4,4'-dibenzofuran-3,3'-diyl]-bis(diphenylphosphinoxid) (VI)

Eine Mischung aus 6,23 g (4-Iod-dibenzofuran-3-yl)-diphenyl-phosphinoxid, 2,39 g Cu-Pulver und 28 ml DMF werden 16 h bei 140°C intensiv unter Luftausschluß gerührt. Die erkaltete Reaktionslösung wird über Celite filtriert, vom Lösungsmittel befreit und mit tert.-Butylmethylether ausgerührt.
Es bleiben 4,08 g Kristalle (88 % d.Th.)
Fp.: 292-300°C (Zers.)
³¹P-NMR ([D₆]-DMSO): 28,6 ppm (S)

### d) Enantiomeren-Trennung

Die Trennung der Enantiomeren erfolgt analog der in ld) beschriebenen Vorgehensweise. Als Elutionsmittel wird ein Gemisch aus Toluol/THF 2:1 (v/v) verwendet. Als erstes eluiert das (-)-Enantiomer.
(S)-(-)-(Bis-4,4'-dibenzofuran-3,3'-diyl)-bis(diphenylphosphinoxid) (VI)
Fp.: 250°C (Modifikations-Änderung), ab 280°C Zersetzung
[α]_{D} = -189° (c=1, DMF)
(R)-(+)-(Bis-4,4'-dibenzofuran-3,3'-diyl)-bis(diphenylphosphinoxid) (VI)
Fp.: 250°C (Modifikations-Änderung), ab 280°C Zersetzung
[α]_{D} = +196° (c=1, DMF)

### e) (S)-(-)-(Bis-4,4'-dibenzofuran-3,3'-diyl)-bis(diphenylphosphin) (I)

Eine Mischung aus 1,0 g (1,36 mol) (S)-(-)-(Bis-4,4'-dibenzofuran-3,3'-diyl)-bis(diphenylphosphinoxid), 6,5 ml Tributylamin, 36 ml Xylol und 1,65 ml Trichlorsilan werden 3 h am Rückfluß gekocht. Bei 0°C werden anschließend 10 ml einer 30 %igen NaOH-Lösung zugegeben. Die Mischung wird mit Methylenchlorid extrahiert.
Nach Trocknung mit gesättigter NaCl-Lösung und MgSO₄ wird durch Chromatographie an Kieselgel (10 % bis 30 % Essigester in Cyclohexan) gereinigt und aus tert.-Butylmethylether ausgerührt.
Ausbeute: 713 mg (75 % d.Th.).
Fp.: 248-250°C
[α]_{D} = -118° (c=1, CHCl₃)
³¹P-NMR (CDCl₃): -13,4 ppm (s)

### f) (R)-(+)-(Bis-4,4'-dibenzofuran-3,3'-diyl)-bis(diphenylphosphin) (I)

Die Reaktion wurde analog e) mit 1 g (R)-(+)-(Bis-4,4'-dibenzofuran-3,3'-diyl)-bis-(diphenylphinoxid) durchgeführt.
Ausbeute: 721 mg (76 % d.Th.)
Fp.: 248-250°C
[α]_{D} = +119° (c=1, CHCl₃)
³¹P-NMR (CDCl₃): -13,4 ppm (s)

### B) Herstellung der Katalysator-Komplexe

### 1) [[(R)-(+)-4,4'-Bis-(dibenzofuran-3,3'-diyl)-bis(diphenylphosphin)]₂Ru₂Cl₄]·NEt₃

Eine Mischung aus 21,4 mg Dichloro-cycloocta-1,5-dien-ruthenium-(II), 59 mg (R)-(+)-(Bis-4,4'-dibenzofuran-3,3'-diyl)-bis(diphenylphosphin), 0,17 ml Triethylamin und 1,7 ml Xylol werden 4 h bei 140°C unter Luftausschluß gerührt.
Anschließend wird das Lösungsmittel im Hochvakuum entfernt.
Ausbeute: quantitativ
³¹P-NMR (CDCl₃): 50,7 (d; J=38,4 Hz); 52,3 (d; J = 38,4 Hz)

### 2) [[(R)-(-)-6,6'-Dichlor-biphenyl-2,2'-diyl)-bis-diphenylphosphin-1,1'-diphenyl]₂Ru₂Cl₄]·NEt₃

Eine Mischung aus 7,5 mg Dichloro-cycloocta-1,5-dien-ruthenium (II), 17,4 mg (R)-6,6'-Dichlorbiphenyl-2,2'-diyl)-bis-diphenylphosphin, 0,06 ml Triethylamin und 2 ml Toluol werden 4 h bei 140°C unter Luftausschluß gerührt. Anschließend wird das Lösungsmittel im Hochvakuum entfernt.
Ausbeute: quantitativ
³¹P-NMR (CDCl₃): 54,8 (d; J = 38 Hz); 55,8 (d; J = 38 Hz)

### 3) [Iodo-Ru-cym-[(R)-(+)-(bis-4,4'-dibenzofuran-3,3'-diyl)-bis-(diphenylphosphin)]]-iodid

Eine Lösung von 34 mg (cym₂Ru₂I₄) in 3 ml Methanol/Methylenchlorid (1:1) wird zu einer Mischung von 50 mg (R)-Bis-4,4'-dibenzofuran-3,3'-diyl)-bis(diphenylphosphin) in 3 ml Methanol/Methylenchlorid (1:1) gegeben, es wird 10 min unter Luftausschluß unter Rückfluß gekocht und eingeengt.
³¹P-NMR (CDCl₃): 41,0 (d; J=57 Hz); 23,5 (d; J=57 Hz)

### 4) [Iodo-Ru-cym-[(R)-(-)-6,6'-dichlor-biphenyl-2,2'-diyl)-bis-diphenylphosphin]]-iodid

Eine Lösung von 42 mg (cym₂Ru₂I₄) in 3 ml Methanol/Methylenchlorid (1:1) wird zu einer Mischung von 34 mg (R)-6,6'-Dichlor-biphenyl-2,2'-diyl)-bis-diphenylphosphin in 3 ml Methanol/Methylenchlorid (1:1) gegeben, es wird 10 min unter Luftausschluß unter Rückfluß gekocht und eingeengt.
³¹P-NMR (CDCl₃): 43,5 (d; J=61 Hz); 26,4 (d; J=61 Hz)

### 5) [(-)-Bis-4,4'-dibenzofuran-3,3'-diyl)-bis-(diphenyl-phosphin)-Ru(OAc)₂]

211 mg des auf gleiche Weise wie im Beispiel B1 hergestellten Katalysators {[((-)-Bis-4,4'-dibenzofuran-3,3'-diyl)-bis-(diphenyl-phosphin))₂Ru₂Cl₄]NEt₃} und 50.5 mg NaOAc wurden in 12 ml entgastem tert.-Butanol unter Luftausschluß unter Rückfluß 12 h gekocht. Das Lösungsmittel wurde unter vermindertem Druck abdestilliert und der Rückstand wurde zweimal mit je 7 ml entgastem Diethylether extrahiert und gefiltert. Die vereinigten Extrakte wurden vom Lösungsmittel befreit und der Rückstand wurde direkt für Hydrierungen eingesetzt.
³¹P-NMR (CDCl₃): 64 ppm (s)

### C) Anwendungsbeispiele

### 1) Hydrierung von 2-(3-Benzyl-phenyl)-propensäure

Eine Lösung von 1 g 2-(3-Benzyl-phenyl)-propensäure und 460 mg Triethylamin in 15 ml entgastem Methanol wird unter Luftausschluß bereitet und mit 21 mg des in Beispiel B1 hergestellten Katalysators versetzt. Anschließend wird 48 h bei 100 atm bei Raumtemperatur hydriert.
Ausbeute: quantitativ
Enantiomerenüberschuß: 89 % e.e. ((-)-Form)
(Die Bestimmung des Enantiomerenüberschusses erfolgt nach Oxidation zu 2-(3-Benzoyl-phenyl)-propionsäure durch HPLC an einer chiralen Phase wie in EP-A-529 444 beschrieben)

### 2) Hydrierung von 2-(3-Benzyl-phenyl)-propensäure

Eine Lösung von 1 g 2-(3-Benzyl-phenyl)-propensäure in 460 mg Triethylamin in 15 ml entgastem Methanol wird unter Luftausschluß mit der Hälfte des im Beispiel B2 bereiteten Katalysators versetzt. Anschließend wird 48 h bei 90 atm bei RT hydriert.
Umsatz und Ausbeute: quantitativ
Enantiomerenüberschuß: 84,2 % e.e ((-)-Form)
(Die Bestimmung des Enantiomerenüberschusses erfolgt durch HPLC wie in Anwendungsbeispiel 1 beschrieben)

### 3) Hydrierung von Acetessigsäuremethylester

1,0 g Acetessigsäuremethylester werden in 15 ml sauerstofffreiem MeOH/CH₂Cl₂ (1:1) gelöst. Anschließend wird die Hälfte des im Beispiel B3 hergestellten Katalysators zugegeben und die Lösung wird in einem mit Argon gespülten Autoklaven transferiert. Anschließend wird 2 Tage bei Raumtemperatur und 90 atm Wasserstoffdruck hydriert. Zur Aufarbeitung wird das Lösungsmittel entfernt.
Umsatz (¹H-NMR): 100 %
Bestimmung des Enantiomerenüberschusses:
Eine Mischung aus 20 mg des Produktes aus Beispiel C3, 1 ml CH₂Cl₂ abs., 50 mg LAC (+)-Methoxyphenyl-trifluormethylessigsäurechlorid und 0,26 ml Pyridin wird über Nacht bei Raumtemperatur gerührt und gaschromatographisch analysiert:
Enantiomerenüberschuß: 98,3 % e.e. ((-)-Form).

### 4) Hydrierung von 2-(3-Benzyl-phenyl)-propensäure

Eine Lösung von 1 g 2-(3-Benzyl-phenyl)-propensäure in 15 ml entgastem Methanol wird unter Luftausschluß bereitet und mit der Hälfte des Produktes von Beispiel B4 versetzt. Anschließend wird 48 h bei 100 atm bei Raumtemperatur hydriert.
Ausbeute und Umsatz: quantitativ
Enantiomerenüberschuß: 83 % e.e. ((-)-Form)
(Die Bestimmung des Enantiomerenüberschusses erfolgt wie unter Beispiel C1 beschrieben)

### 5) Hydrierung von 2-(3-Benzyl-phenyl)-propensäure

Eine Lösung von 1 g 2-(3-Benzyl-phenyl)-propensäure und 460 mg Triethylamin in 15 ml entgastem Methanol wird unter Luftausschluß mit einem Drittel des in Beispiels B3 bereiteten Katalysators versetzt. Anschließend wird 48 h bei 90 atm bei RT hydriert.
Umsatz und Ausbeute: quantitativ
Enantiomerenüberschuß: 87 % e.e.((-)-Form)

### 6) Hydrierung von 2-(3-Benzyl-phenyl)-propensäure

Zu einer Lösung von 1 g 2-(3-Benzyl-phenyl)-propensäure und 460 mg Triethylamin in 15 ml entgastem Methanol wird unter Luftausschluß 30 mg des im Beispiel B5 hergestellten Katalysators zugegeben. Die Mischung wird dann 60 h bei RT mit 90 atm Wasserstoff hydriert.
Umsatz und Ausbeute: quantitativ
Enantiomerenüberschuß: 87,6 % e.e. ((+)-Form)

### 7) Hydrierung von 2-(3-Benzyl-phenyl)-propensäure mit in situ gebildeten [Ru((-)-Bis-4,4'-dibenzofuran-3,3'-diyl)-bis-(diphenyl-phosphin))(acac)₂]

Eine Lösung von 22 mg Ru(acac)₃, 43 mg (-)-Bis-4,4'-dibenzofuran-3,3'-diyl)-bis-(diphenyl-phosphin), 2 g 2-(3-benzyl-phenyl)-propensäure und 0,92 g Triethylamine in 30 ml entgastem Methanol wird 48 bei RT und 90 atm Wasserstoff hydriert.
Umsatz und Ausbeute: quantitativ
Enantiomerenüberschuß: 83,0 % e.e. ((+)-Form)

## Patentansprüche

1. Enantiomerenreine Bisphosphine der allgemeinen Formel (I) 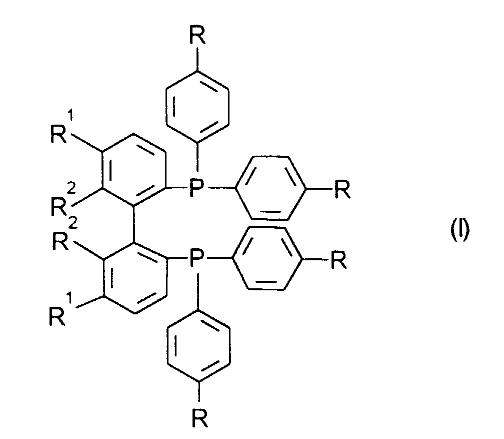 in welcher
R für Wasserstoff oder Alkyl mit 1 bis 4 C-Atomen steht,
R¹ für Wasserstoff steht und
R² für Chlor steht oder
R¹ und R² gemeinsam für den Rest der Formel 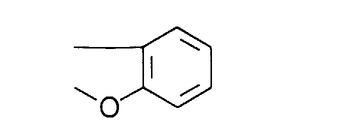 stehen, in ihrer (R)- oder (S)-Form.

2. Bisphosphine der allgemeinen Formel (I) gemäß Anspruch 1, in welcher
R und R¹ für Wasserstoff stehen,
R² für Chlor steht
oder
R¹ und R² gemeinsam für den Rest der Formel 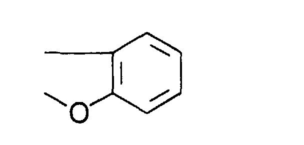 stehen.

3. Verfahren zur Herstellung von Bisphosphinen der allgemeinen Formel (I) gemäß Anspruch 1, dadurch gekennzeichnet, daß man Halogenphenylverbindungen der allgemeinen Formel (II) 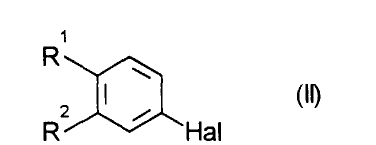 in welcher
R¹ und R² die oben angegebene Bedeutung haben und
Hal für Halogen, insbesondere für Brom steht,
mit Diphenylphosphoroxychlorid der allgemeinen Formel (III) 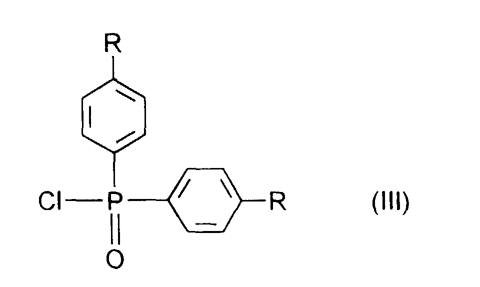 in welcher
R die oben angegebene Bedeutung hat,
nach üblichen Methoden z.B. über eine Grignard-Reaktion zu Verbindungen der allgemeinen Formel (IV) 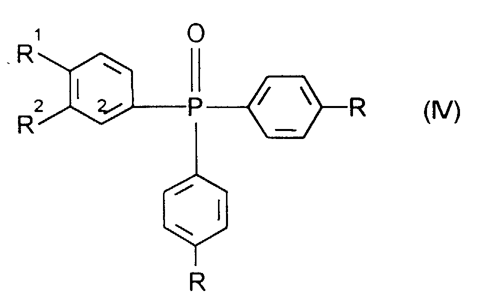 in welcher
R, R¹ und R² die oben angegebene Bedeutung haben,
umsetzt und diese mit Lithium in der 2-Position metalliert und anschließend mit Iod zu Verbindungen der Formel (V) 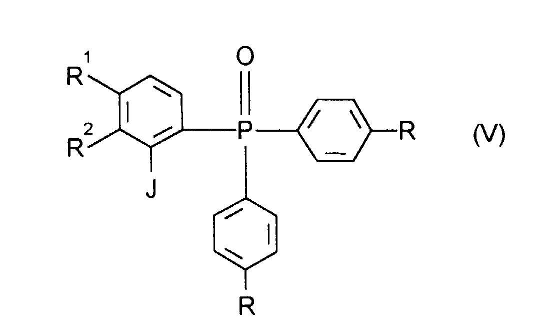 umsetzt, nach üblichen Methoden (Ullmann-Kupplung) zu racemischen Verbindungen der allgemeinen Formel (VI) 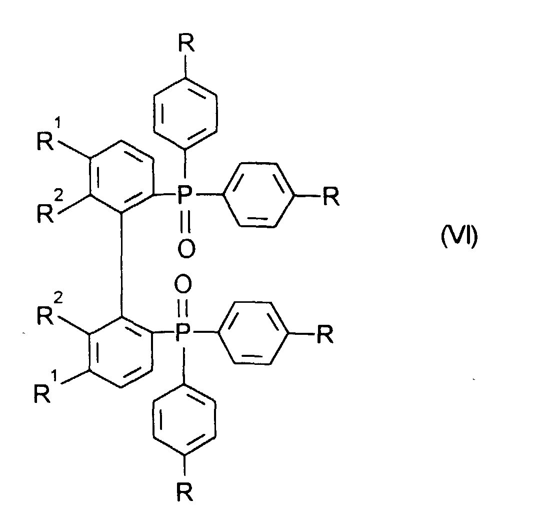 in welcher
R, R¹ und R² die oben angegebene Bedeutung haben,
dimerisiert, und diese Phosphinoxide in ihre Enantiomere auftrennt und anschließend die erhaltenen (S)- oder (R)-Enantiomere nach üblichen Methoden zu Verbindungen der Formel (I) reduziert.

4. Verbindungen der allgemeinen Formel (V) 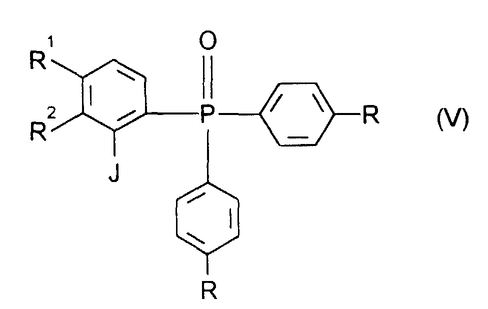 in welcher
R für Wasserstoff oder Alkyl mit 1 bis 4 C-Atomen steht,
R¹ für Wasserstoff steht und
R² für Chlor steht oder
R¹ und R² gemeinsam für den Rest der Formel 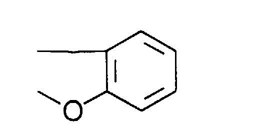 stehen.

5. Verbindungen der allgemeinen Formel (VI) 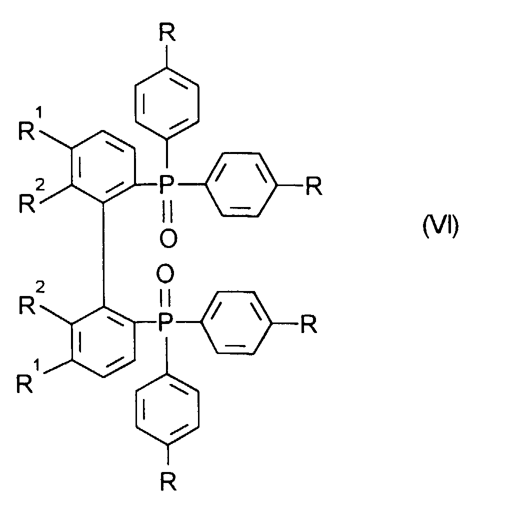 in welcher
R für Wasserstoff oder Alkyl mit 1 bis 4 C-Atomen steht,
R¹ für Wasserstoff steht und
R² für Chlor steht oder
R¹ und R² gemeinsam für den Rest der Formel 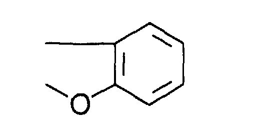 stehen, in ihrer racemischen oder in ihrer (R)- oder (S)-Form.

6. Komplexe von Bisphosphinen der allgemeinen Formel (I) mit einem Metall der Gruppe (VIII).

7. Komplexe von Bisphosphinen der allgemeinen Formel (I) mit Ru, Rh oder Ir.

8. Komplexe von Bisphosphinen der allgemeinen Formel (I) mit Ru.

9. Verwendung von Bisphosphinen der allgemeinen Formel (I) gemäß Anspruch 1 in Form ihrer Komplexe mit Metallen der Gruppe (VIII) bei der Durchführung von asymmetrischen Hydrierungen und für enantioselektive H-Verschiebungen in prochiralen Systemen.

10. Verwendung von Verbindungen der allgemeinen Formel (I) gemäß Anspruch 1 in Form ihrer Komplexe mit Ruthenium bei der asymmetrischen Hydrierung von 2-Arylpropensäuren oder ihrer Salze.

11. Verwendung von Bisphosphinen der allgemeinen Formel (I) gemäß Anspruch 1 in Form ihrer Komplexe mit Ruthenium bei der asymmetrischen Hydrierung von
a) folgenden Ketogruppen zu den entsprechenden Alkoholen,
a1) von substituierten oder unsubstituierten α- oder β-Ketoestern oder α- oder β-Ketoamiden,
a2) von α- oder β-Aminoketonen oder von α- oder β-Hydroxyketonen
oder
b) der C-C-Doppelbindung von Allylaminen, Allylalkoholen oder Acetamidozimtsäure-Derivaten.

## Claims

1. Enantiomerically pure bisphosphines of the general formula (I) 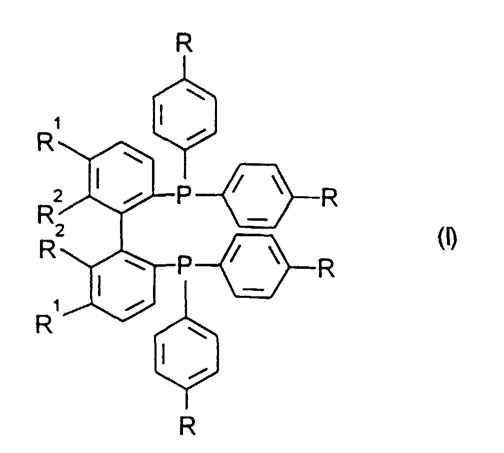 in which
R represents hydrogen or alkyl having from 1 to 4 carbon atoms,
R¹ represents hydrogen and
R² represents chlorine or
R¹ and R² together represent the radical of the formula 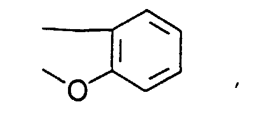 in their (R)- or (S)-form.

2. Bisphosphines of the general formula (I) according to Claim 1, in which
R and R¹ represent hydrogen,
R² represents chlorine
or
R¹ and R² together represent the radical of the formula

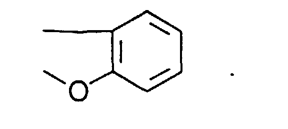

3. Process for preparing bisphosphines of the general formula (I) according to Claim 1, characterized in that halogenophenyl compounds of the general formula (II) 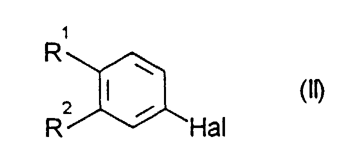 in which
R¹ and R² have the meanings given above and
Hal represents halogen, in particular bromine,
are reacted with diphenylphosphinic chloride of the general formula (III) 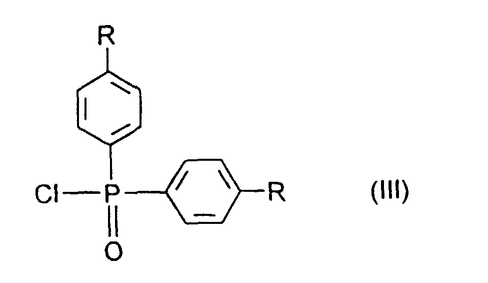 in which
R has the meaning given above,
by conventional methods, for example via a Grignard reaction, to give compounds of the general formula (IV) 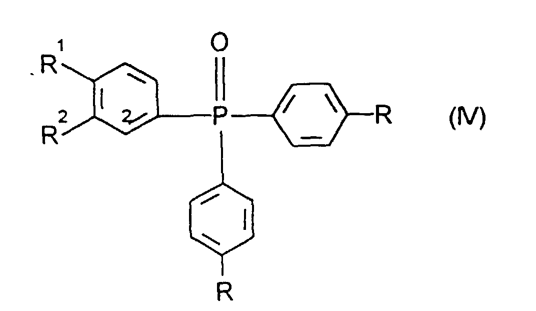 in which
R, R¹ and R² have the meanings given above,
and these are metallated with lithium in the 2-position and subsequently reacted with iodine to give compounds of the formula (V) 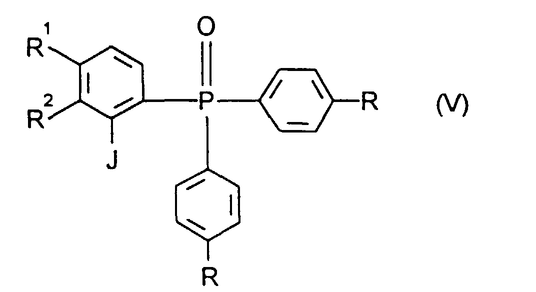 dimerized by conventional methods (Ullmann coupling) to give racemic compounds of the general formula (VI) 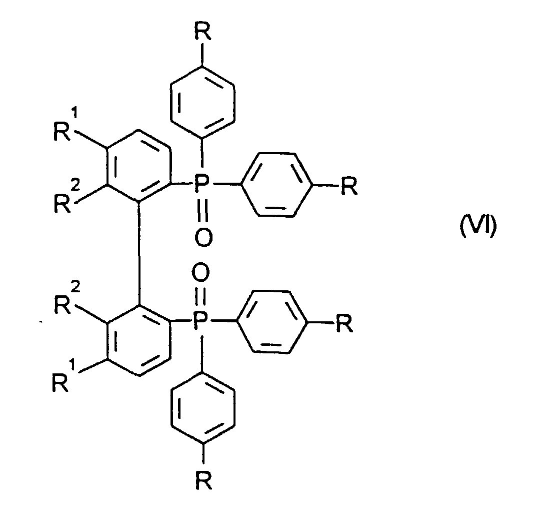 in which
R, R¹ and R² have the meanings given above,
and these phosphine oxides are resolved into their enantiomers and the (S) or (R) enantiomers obtained are subsequently reduced by conventional methods to give compounds of the formula (I).

4. Compounds of the general formula (V) 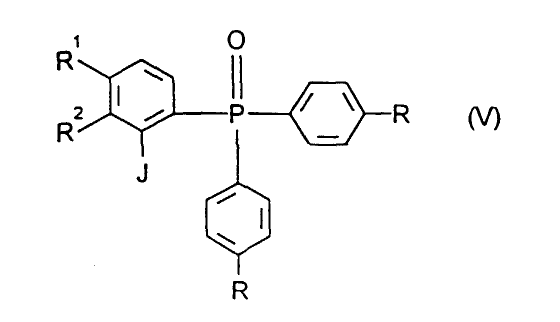 in which
R represents hydrogen or alkyl having from 1 to 4 carbon atoms,
R¹ represents hydrogen and
R² represents chlorine or
R¹ and R² together represent the radical of the formula

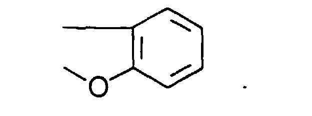

5. Compounds of the general formula (VI) 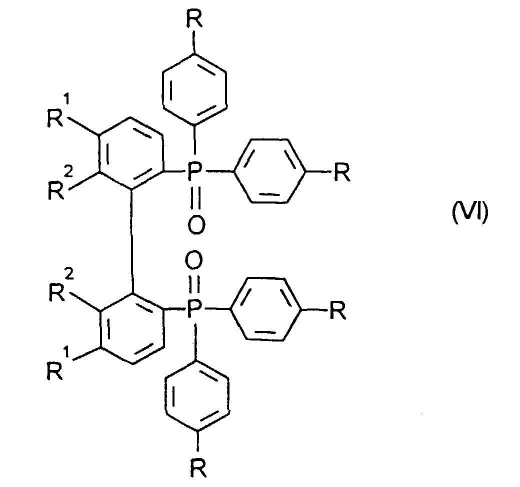 in which
R represents hydrogen or alkyl having from 1 to 4 carbon atoms,
R¹ represents hydrogen and
R² represents chlorine or
R¹ and R² together represent the radical of the formula 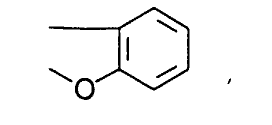 in their racemic or in their (R)- or (S)-form.

6. Complexes of bisphosphines of the general formula (I) with a metal of group (VIII).

7. Complexes of bisphosphines of the general formula (I) with Ru, Rh or Ir.

8. Complexes of bisphosphines of the general formula (I) with Ru.

9. Use of bisphosphines of the general formula (I) according to Claim 1 in the form of their complexes with metals of group (VIII) in carrying out asymmetric hydrogenations and for enantioselective H shifts in prochiral systems.

10. Use of compounds of the general formula (I) according to Claim 1 in the form of their complexes with ruthenium in the asymmetric hydrogenation of 2-arylpropenoic acids or salts thereof.

11. Use of bisphosphines of the general formula (I) according to Claim 1 in the form of their complexes with ruthenium in the asymmetric hydrogenation of
a) the following keto groups to the corresponding alcohols,
a1) of substituted or unsubstituted α- or β-ketoesters or α- or β-ketoamides,
a2) of α- or β-aminoketones or of α- or β-hydroxy-ketones
or
b) the C-C double bond of allylamines, allyl alcohols or acetamidocinnamic acid derivatives.

## Revendications

1. Bisphosphines à l'état d'énantiomères purs, répondant à la formule générale (I) 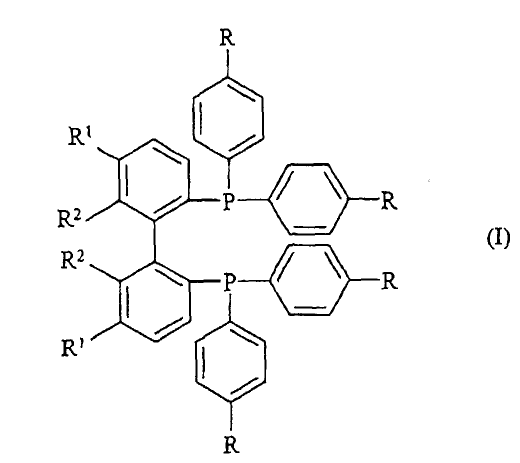 dans laquelle
R représente l'hydrogène ou un groupe alkyle en C₁-C₄,
R¹ représente l'hydrogène,
R² représente le chlore, ou bien
R¹ et R² représentent ensemble le groupe de formule 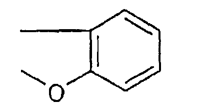 sous leur forme (R) ou (S).

2. Bisphosphines de fonnule générale (I) de la revendication 1, dans laquelle
R et R¹ représentent l'hydrogène,
R² représente le chlore,
ou bien
R¹ et R² représentent ensemble le groupe de formule

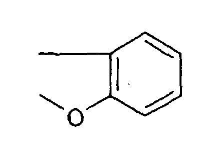

3. Procédé de préparation des bisphosphines de formule générale (I) de la revendication 1, caractérisé en ce que l'on fait réagir des dérivés halogénophényliques de formule générale (II) 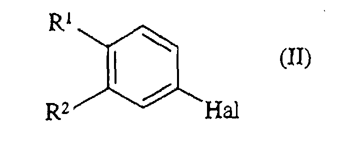 dans laquelle
R¹ et R² ont les significations indiquées ci-dessus et
Hal représente un halogène, plus spécialement le brome,
avec l'oxychlorure de diphénylphosphine de formule générale (III) 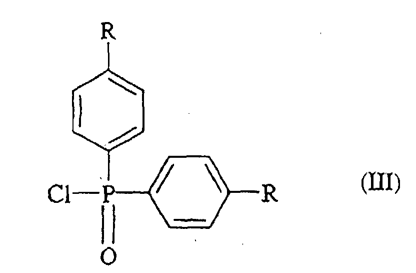 dans laquelle
R a les significations indiquées ci-dessus,
selon des modes opératoires usuels, par exemple dans une réaction de Grignard, ce qui donne les composés de formule générale (IV) 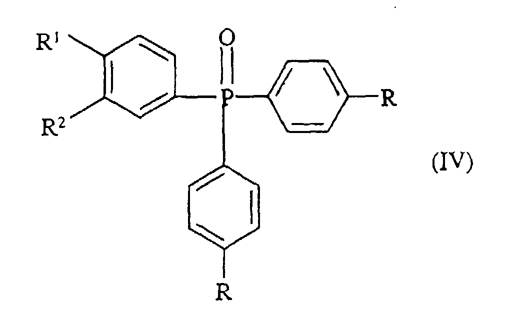 dans laquelle
R, R¹ et R² ont les significations indiquées ci-dessus,
qu'on métallise par le lithium en position 2, après quoi on fait réagir avec l'iode, ce qui donne les composés de formule (V) 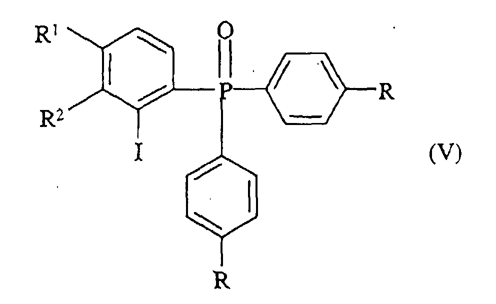 qu'on dimérise par des modes opératoires usuels (condensation d'Ullmann) en les composés racémiques de formule générale (VI) 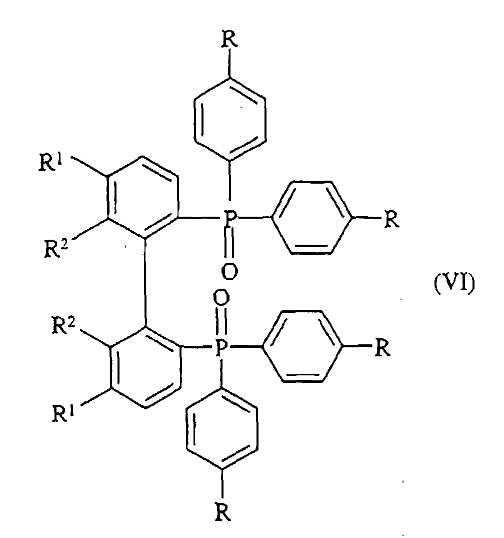 dans laquelle
R, R¹ et R² ont les signification indiquées ci-dessus,
ces oxydes de phosphines étant ensuite séparés en leurs énantiomères, après quoi on convertit les énantiomères S ou R obtenus, selon des modes opératoires usuels en composés de formule (I).

4. Composés de formule générale (V) 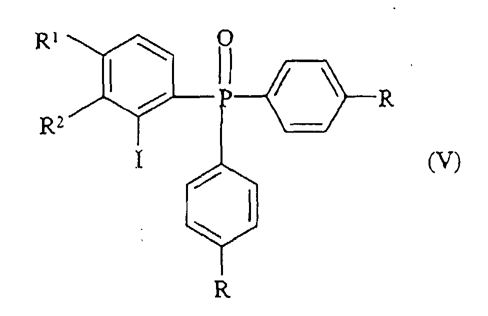
R représente l'hydrogène ou un groupe alkyle en C₁-C₄,
R¹ représente l'hydrogène et
R² représente le chlore, ou bien
R¹ et R² représentent ensemble le groupe de formule

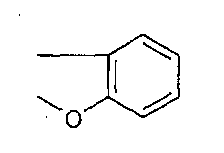

5. Composés de formule générale (VI) 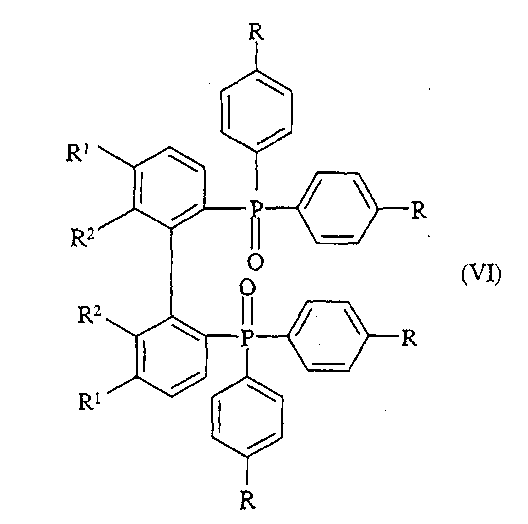 dans laquelle
R représente l'hydrogène ou un groupe alky]e en C₁-C₄,
R¹ représente l'hydrogène et
R² représente le chlore, ou bien
R¹ et R² représentent ensemble le groupe de formule 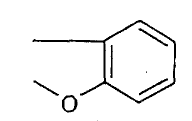 sous leur forme racémique ou sous leur forme (R) ou (S).

6. Complexes des bisphosphines de formule générale (I) et d'un métal du groupe VIII.

7. Complexes des bisphosphines de formule générale (I) et de Ru, Rh ou Ir.

8. Complexes des bisphosphines de formule générale (I) et de Ru.

9. Utilisation des bisphosphines de formule générale (I) de la revendication 1 à l'état de complexes de métaux du groupe VIII pour des hydrogénations asymétriques et pour des déplacements énantiosélecfifs de l'hydrogène dans des systèmes prochiraux.

10. Utilisation des composés de formule générale (I) de la revendication 1 à l'état de complexes du ruthénium pour l'hydrogénation asymétrique des acides 2-arylpropénoïques ou de leurs sels.

11. Utilisation des bisphosphines de formule générale (I) de la revendication 1 à l'état de complexes du ruthénium pour l'hydrogénation asymétrique de
a) les groupes céto des composés suivants en les alcools correspondants:
a1) les α- ou β-cétoesters ou α- ou β-cétoamides substitués ou non substitués,
a2) les α- ou β-aminocétones ou α- ou β-hydroxycétones
ou bien
b) la double liaison C-C des allylamines, des alcools allyliques ou des dérivés de l'acide acétamidocinnamique.
